# EUROPÄISCHE PATENTSCHRIFT

(11) **EP 1 763 506 B1**
(45) Veröffentlichungstag und Bekanntmachung des Hinweises auf die Patenterteilung: **15.10.2008**
(21) Anmeldenummer: 05755967.6
(22) Anmeldetag: 04.06.2005
(51) Int. Cl.: C07C 253/00, C07C 255/03, C07C 255/32

(54) **VERFAHREN ZUR HERSTELLUNG VON NITRILEN DURCH ELIMINIERUNG VON WASSER AUS ALDEHYDOXIMEN MIT ALKYLPHOSPHONSÄUREANHYDRIDEN**
METHOD FOR PRODUCING NITRILES BY ELIMINATION OF WATER FROM ALDEHYDE OXIMES WITH ALKYLPHOSPHONIC ANHYDRIDES
PROCEDE POUR PRODUIRE DES NITRILES PAR ELIMINATION D'EAU D'ALDEHYDOXIMES AVEC DES ANHYDRIDES D'ACIDE D'ALKYLPHOSPHONE

(30) Priorität: 19.06.2004 DE 102004029812
(43) Veröffentlichungstag der Anmeldung: 21.03.2007
(73) Patentinhaber: Archimica GmbH, 65929 Frankfurt am Main (DE)
(72) Erfinder: MEUDT, Andreas, 65719 Hofheim (DE); SCHERER, Stefan, 64347 Griesheim (DE); BÖHM, Claudius, 60594 Frankfurt am Main (DE)
(74) Vertreter: Schweitzer, Klaus
(86) Internationale Anmeldenummer: PCT/EP2005/006015
(87) Internationale Veröffentlichungsnummer: WO 2005/123661

(56) Entgegenhaltungen:
- EP-A- 0 550 762
- WO-A-98/05630

## Beschreibung

Nitrile sind bedeutsame und äußerst vielseitig verwendbare Zwischenprodukte in der organischen Synthese. Diese Verbindungsklasse zeigt eine hohe Reaktivität der C,N-Dreifachbindung, wodurch zahlreiche Additionsreaktionen ermöglicht werden. Die Bedeutung in der modernen organischen Synthese wird nur durch Limitierungen der Zugänglichkeit dieser Verbindungsklassen eingeschränkt.

Standardverfahren zur Herstellung von Nitrilen (WO98/05630) sind Eliminierungen entsprechend funktionalisierter Aldoxime. Dabei kommen zahllose Methoden, u.a. unter Verwendung von Essigsäureanhydrid, Essigsäurechlorid, Thionylchlorid oder Bleioxid zum Einsatz. Ferner sind als wasserabspaltende Mittel noch Phosphorpentoxid, Benzolsulfonylchlorid, Chlorameisensäureethylester oder wässrige Alkalien benutzt worden. Eine thermische Abspaltung bei 340 - 360°C am Metalloxidkontakt ist ebenfalls möglich. Auch Eintopf-Verfahren ausgehend von Aldehyden finden Verwendung. Allerdings muss dabei stets bei 70 - 140°C gearbeitet werden. Bei 20°C findet die Umwandlung von Aldehyden mit Hydroxylamin-Hydrochlorid und Trifluoressigsäureanhydrid statt. Eine andere Variante ist die Umsetzung von Aldehyden mit Hydroxylamin-Hydrochlorid in Pyridin/Wasser in Gegenwart von Dicyclohexylcarbodiimid, Cu(II)-Salz und Triethylamin. Dabei fällt neben Schwermetallsalzen auch nur äußerst schwierig abtrennbarer Dicyclohexylharnstoff an.

In der modernen organischen Synthese nimmt die Bedeutung chemo-, regio- und stereoselektiver Reagenzien explosionsartig zu. Will man beispielsweise aus einem komplexen Molekül mit zahlreichen funktionellen Gruppen ein Oxim in ein Nitril überführen, so scheiden zahlreiche der genannten Methoden aus Selektivitätsgründen aus. Auch die Verwendung der thermischen Dehydratisierung ist eingeschränkt, da sehr hohe Temperaturen von bis zu 450°C benötigt werden.

Eine selektive und bevorzugte Methode zur Eliminierung von Oximen zu Nitrilen ist die Reaktion mit einer Vielzahl von wasserentziehenden Mittel.

Eine hochselektive Problemlösung für die genannten Transformationen fehlte bisher. Die bekannten Reagenzien können zwar die gewünschten Transformationen bewerkstelligen, dabei werden aber oft andere Gruppierungen ebenfalls beeinflusst. In vielen Fällen werden durch die erforderlichen drastischen Bedingungen selbst entfernt stehende Stereozentren epimerisiert. Zusätzlich sollte die Transformation daher bei sehr milden Bedingungen anwendbar und besonders die Abtrennung der Folgeprodukte des eingesetzten Reagenzes sehr einfach sein.

Aufgabe ist es daher ein wirtschaftliches Verfahren bereitzustellen, dass es erlaubt Oxime durch Eliminierung von Wasser in die entsprechenden Nitrile zu überführen, dabei aber gleichzeitig die Anwendung sehr milder Reaktionsbedingungen gestattet und eine vereinfachte Aufarbeitung aufweist.

Die im Stand der Technik bekannten Verfahren zur Herstellung von Nitrilen weisen alle gravierenden Nachteile auf:
So muss bei der Verwendung von Thionylchlorid die Reaktion bei erhöhter Temperatur durchgeführt werden. Das ist vor allem bei empfindlichen
Verbindungen nachteilig. Bei der Verwendung von Bleioxid fällt das Schwermetall im Abfallstrom an.
Zwar hat sich in der einstufigen Darstellung von Nitrilen, ausgehend von Aldehyden, das Voswinkel-Verfahren bewährt, aber die Verwendung von Dicyclohexylcarbodiimid birgt Nachteile, da dies sensibilisierend ist und die Abtrennung der Reaktionsnebenprodukte oft nicht vollständig gelingt.

Überraschenderweise wurde gefunden, dass der Einsatz von cyclischen 2,4,6-substituierten 1,3,5,2,4,6-trioxatriphosphinan-2,4,6-trioxiden alle diese Probleme löst. Diese Eliminierungsmethode ermöglicht die hochselektive Überführung von Oximen in die entsprechenden Nitrile, wobei gleichzeitig die gewünschte Epimerisierungsfreiheit und maximale Regio- und Stereoselektivität bei zugleich nahezu quantitativen Ausbeuten beobachtet wird.

Die vorliegende Erfindung betrifft somit ein hochselektives Verfahren zur Herstellung von Nitrilen der Formel (II)

R¹-CN (II)

durch Umsetzung von
Aldehydoximen (R¹CH = N - OH)
mit cyclischen Alkylphosphonsäureanhydriden und gegebenenfalls einer Aminbase NR₃², bei einer Temperatur im Bereich von -100 bis +120°C,
wobei R¹ für H, einen linearen oder verzweigten, substituierten oder unsubstituierten C₁-C₁₂-Alkylrest, einen C₃-C₁₀ Cycloalkyl-, Alkenyl- oder einen Aryl- oder Heteroarylrest steht.

In einer bevorzugten erfindungsgemäßen Ausführungsform ist das cyclische Alkylphosphonsäureanhydrid ein 2,4,6-substituiertes 1,3,5,2,4,6-trioxatriphosphinan-2,4,6-trioxid der Formel (I), 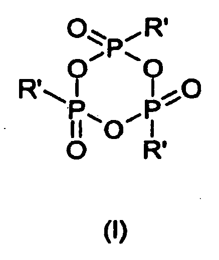 worin R' unabhängig voneinander für Allyl, Aryl oder offenkettige oder verzweigte C₁ bis C₁₂-Alkyl-Reste, insbesondere für C₁-C₈-Alkylreste steht.

Besonders bevorzugt werden Phosphonsäureanhydride der Formel (I) in denen R' für einen Methyl, Ethyl, n-Propyl, Isopropyl, n-Butyl, 2-Butyl, Isobutyl, Pentyl, Hexyl, insbesondere einen Ethyl-, Propyl- und/oder Butyl-Rest steht.

Die Eliminierung zu Nitrilen (II) kann dabei im allgemeinen bei Temperaturen im Bereich von -100 bis +120°C durchgeführt werden, bevorzugt sind Temperaturen im Bereich von -30 bis +30°C, wobei tiefere Temperaturen im allgemeinen mit höheren Selektivitäten korreliert sind. Die Reaktionsdauer ist abhängig von der angewandten Temperatur und beträgt im allgemeinen 1 bis 12 Stunden, insbesondere 3 bis 6 Stunden.

Der Zusatz von Aminen ist im Allgemeinen nicht erforderlich, kann sich aber im Einzelfall als vorteilhaft erweisen. Als Amine werden im Allgemeinen Amine der Formel (III)

NR²₃ (III)

eingesetzt, wobei R² für H, Allyl, Aryl oder offenkettige, cyclische oder verzweigte C₁ bis C₁₂-Alkyl-Reste, Aryloxy, Allyloxy oder Alkoxy mit offenkettigen, cyclischen oder verzweigten C₁ bis C₁₂-Alkyl-Resten oder eine Kombination aus den genannten Substituenten steht.

Besonders bevorzugt werden Amine der Formel (III) in denen R² für ein H, Methyl, Ethyl, n-Propyl, Isopropyl, n-Butyl, 2-Butyl, Isobutyl, Pentyl, Hexyl, Phenyl insbesondere ein H, Ethyl, n-Propyl, Isopropyl, n-Butyl, 2-Butyl, Isobutyl oder Phenyl oder eine Kombination aus den genannten Substituenten steht.

Das cyclische Phosphonsäureanhydrid kann dem Reaktionsmedium entweder als Schmelze oder als flüssige Mischung gelöst in einem Lösungsmittel zugegeben werden.
Geeignete Lösungsmittel sind dabei solche, die keine Nebenreaktionen mit dem Phosphonsäureanhydrid ergeben, dies sind alle aprotischen organischen Lösungsmittel, wie z.B. Ligroin, Butan, Pentan, Hexan, Heptan, Octan, Cyclobutan, Cyclopentan, Cyclohexan, Cycloheptan, Cyclooctan, Dichlormethan, Chloroform, Tetrachlorkohlenstoff, 1,2-Dichlorethan, 1,1,2,2-Tetrachlorethan, Methylacetat, Ethylacetat, Propylacetat, Butylacetat, Dimethylformamid, Diethylformamid, Dimethylacetamid, Diethylacetamid, Diethylether, Diisopropylether, tert-Butyl-Methylether, THF, Dioxan, Acetonitril oder Gemische aus diesen, besonders bevorzugt sind Dichlormethan, Chloroform, Ethylacetat, Propylacetat, Butylacetat, Dimethylformamid, Diethylformamid, Dimethylacetamid, Diethylacetamid, Diisopropylether, tert-Butyl-Methylether, THF, Dioxan, Acetonitril oder Gemische aus diesen, ganz besonders bevorzugt werden Dichlormethan, Chloroform, Ethylacetat, Butylacetat, Dimethylacetamid, tert-Butyl-Methylether, THF, Dioxan, Acetonitril oder Gemische aus diesen, insbesondere bevorzugt sind THF, Ethylacetat oder Butylacetat.

Die Zugabe des Phosphonsäureanhydrids erfolgt im Allgemeinen mindestens drittelstöchiometrisch im Bezug auf die Ausgangsverbindung, kann aber auch überstöchiometrisch sein, beispielsweise im Verhältnis 1 Ausgangsverbindung : 1,2 T3P^{®} (cyclisches Propanphosphonsäureanhydrid).

Die Umsetzungen werden bevorzugt so durchgeführt, dass die entsprechende Ausgangsverbindung bei der Reaktionstemperatur zu T3P^{®} in einem geeigneten Lösungsmittel gegeben wird.

Die Isolierung des Reaktionsproduktes erfolgt bevorzugt durch Hydrolyse und einfache Phasentrennung, da die Folgeprodukte der Phosphonsäureanhydride allgemein sehr gut wasserlöslich sind. Je nach Natur des zu isolierenden Produkts können dabei auch Nachextraktionen erforderlich sein. Das gebildete Phosphonsäureanhydrid-Folgeprodukt stört Folgereaktionen oft nicht, so dass auch der direkte Einsatz der erhaltenen Reaktionslösungen oft sehr gute Ergebnisse bringt.
Alle benannten Verfahrensweisen zeichnen sich durch sehr gute Ausbeuten (typisch 90-100 %, insbesondere > 95 %) bei gleichzeitiger Abwesenheit von Nebenreaktionen und Epimerisierungen aus. Die Selektivitäten der erfindungsgemäßen Reaktion liegen im Bereich von 97-100 %, insbesondere 99-100 %.

Das erfindungsgemäße Verfahren soll durch das nachfolgende Beispiel erläutert werden, ohne die Erfindung darauf zu beschränken:

### Beispiel 1: Eliminierung von Benzaldehydoxim zu Benzonitril

1 mol T3P^{®} in Ethylacetat (50 % w/w) wird auf 0°C gekühlt. Zu dieser Lösung wird Benzaldehydoxim getropft und 3 Stunden bei dieser Temperatur nachgerührt. Zu dieser Zeit zeigte das Reaktions-GC einen Umsatz von 100 % an. Nach Erwärmen auf Raumtemperatur wurden 180 ml Wasser zugeben und die Phasen getrennt. Nach Abkondensieren des Lösungsmittels verblieb das Benzonitril in einer Ausbeute von 97 %, GC-Reinheit 99 % (a/a).

### Beispiel 2: Eliminierung von Furfuraloxim zu 2-Cyanofuran

1 mol T3P^{®} in Ethylacetat (50 % w/w) wird auf 0°C gekühlt. Zu dieser Lösung wird Furfuraloxim getropft und 3 Stunden bei dieser Temperatur nachgerührt. Zu dieser Zeit zeigte das Reaktions-GC einen Umsatz von 100 % an. Nach Erwärmen auf Raumtemperatur wurden 180 mL Wasser zugeben und die Phasen getrennt. Nach Abkondensieren des Lösungsmittels verblieb das 2-Cyanofuran in einer Ausbeute von 97 %, GC-Reinheit 99 % (a/a).

### Beispiel 3: Eliminierung von Octanaloxim zu Octannitril

1 mol T3P^{®} in Ethylacetat (50 % w/w) wird auf 0°C gekühlt. Zu dieser Lösung wird Octanaloxim getropft und 3 Stunden bei dieser Temperatur nachgerührt. Zu dieser Zeit zeigte das Reaktions-GC einen Umsatz von 100 % an. Nach Erwärmen auf Raumtemperatur wurden 180 mL Wasser zugeben und die Phasen getrennt. Nach Abkondensieren des Lösungsmittels verblieb das Octannitril in einer Ausbeute von 97 %, GC-Reinheit 99 % (a/a).

## Patentansprüche

1. Verfahren zur Herstellung von Nitrilen der Formel (II)
R¹ - CN (II)
durch Umsetzung von
Aldehydoximen (R¹CH = N - OH)
mit cyclischen Alkylphosphonsäureanhydriden bei einer Temperatur im Bereich von -100 bis +120°C
wobei R¹ für H, einen linearen oder verzweigten C₁-C₁₂-Alkylrest, einen C₃-C₁₀ Cycloalkyl-, Alkenyl- oder einen Aryl- oder Heteroarylrest steht.

2. Verfahren nach Anspruch 1, **dadurch gekennzeichnet, dass** das cyclische Alkylphosphonsäureanhydrid ein 2,4,6-substituiertes 1,3,5,2,4,6-trioxatriphosphinan-2,4,6-trioxid der Formel (I) ist, 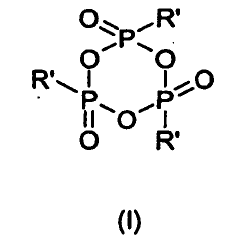 worin R' unabhängig voneinander für Allyl, Aryl oder offenkettige oder verzweigte C₁ bis C₁₂-Alkyl-Reste steht.

3. Verfahren nach Anspruch 2, **dadurch gekennzeichnet, dass** R' für einen Methyl, Ethyl, n-Propyl, Isopropyl, n-Butyl, 2-Butyl, Isobutyl, Pentyl, Hexyl, insbesondere einen Ethyl-, Propyl- und/oder Butyl-Rest steht.

4. Verfahren nach mindestens einem der vorhergehenden Ansprüche,
**dadurch gekennzeichnet, dass** die Umsetzung in Gegenwart einer Aminbase der Formel (III) durchgeführt wird
NR²₃ (III)
eingesetzt, wobei R² für H, Allyl, Aryl oder offenkettige, cyclische oder verzweigte C₁ bis C₁₂-Alkyl-Reste, Aryloxy, Allyloxy oder Alkoxy mit offenkettigen, cyclischen oder verzweigten C₁ bis C₁₂-Alkyl-Resten oder eine Kombination aus den genannten Substituenten steht.

5. Verfahren nach mindestens einem der vorhergehenden Ansprüche, **dadurch gekennzeichnet, dass** das cyclische Alkylphosphonsäureanhydrid entweder als Schmelze oder gelöst in einem Lösungsmittel der Reaktionslösung zugegeben wird.

6. Verfahren nach Anspruch 5, **dadurch gekennzeichnet, dass** das cyclische Alkylphosphonsäureanhydrid in einem aprotischen Lösungsmittel zugegeben wird.

7. Verfahren nach mindestens einem der vorhergehenden Ansprüche, **dadurch gekennzeichnet, dass** die Reaktionslösung vor Zugabe des Alkylphosphonsäureanhydrid auf die Reaktionstemperatur temperiert wird.

8. Verfahren nach mindestens einem der vorhergehenden Ansprüche, **dadurch gekennzeichnet, dass** das Alkylphosphonsäureanhydrid in bezug auf die Ausgangsverbindung drittelstöchiometrisch bis überstöchiometrisch eingesetzt wird.

## Claims

1. Process for preparing nitriles of the formula (II)
R¹-CN (II)
by reacting
aldehyde oximes (R¹CH = N-OH)
with cyclic alkylphosphonic anhydrides at a temperature in the range from -100 to +120 °C,
where R¹ is H, a linear or branched C₁-C₁₂-alkyl radical, a C₃-C₁₀-cycloalkyl radical, alkenyl radical or an aryl or heteroaryl radical.

2. The process as claimed in claim 1, wherein the cyclic alkylphosphonic anhydride is a 2,4,6-substituted 1,3,5,2,4,6-trioxatriphosphinane 2,4,6-trioxide of the formula (I) 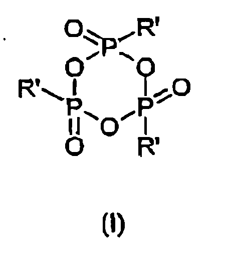 where R' is independently allyl, aryl or open-chain or branched C₁ to C₁₂-alkyl radicals.

3. The process as claimed in claim 2, wherein R' is a methyl, ethyl, n-propyl, isopropyl, n-butyl, 2-butyl, isobutyl, pentyl, hexyl, especially an ethyl, propyl and/or butyl radical.

4. The process as claimed in at least one of the preceding claims, wherein the reaction is performed in the presence of an amine base of the formula (III)
NR²₃ (III)
where R² is H, allyl, aryl or open-chain, cyclic or branched C₁ to C₁₂-alkyl radicals, aryloxy, allyloxy or alkoxy having open-chain cyclic or branched C₁ to C₁₂-alkyl radicals, or a combination of the substituents mentioned.

5. The process as claimed in at least one of the preceding claims, wherein the cyclic alkylphosphonic anhydride is added to the reaction solution either as a melt or dissolved in a solvent.

6. The process as claimed in claim 5, wherein the cyclic alkylphosphonic anhydride is added in an aprotic solvent.

7. The process as claimed in at least one of the preceding claims, wherein the reaction solution is heated to the reaction temperature before the alkylphosphonic anhydride is added.

8. The process as claimed in at least one of the preceding claims, wherein the alkylphosphonic anhydride is used in one third of the stoichiometric amount up to a superstoichiometric amount in relation to the starting compound.

## Revendications

1. Procédé pour la préparation de nitriles de formule (II)
R¹-CN (II)
par la réaction d'aldéhyde-oximes (R¹CH=N-OH) avec des anhydrides d'acides alkylphosphoniques à une température dans la plage allant de -100 à +120 °C.
R¹ représentant H, un radical alkyle en C₁-C₁₂ linéaire ou ramifié, un radical alcényle, cycloalkyle en C₃-C₁₀ ou un radical aryle ou hétéroaryle.

2. Procédé selon la revendication 1, **caractérisé en ce que** l'anhydride d'acide alkylphosphonique cyclique est un 1,3,5,2,4,6-trioxatriphosphinane-2,4,6-trioxyde substitué en 2,4,6 de formule (I), 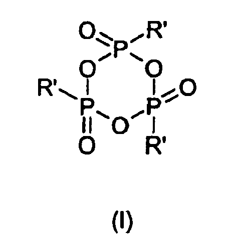 dans lequel les radicaux R' représentent chacun indépendamment un radical allyle, aryle ou alkyle en C₁-C₁₂ linéaire ou ramifié.

3. Procédé selon la revendication 2, **caractérisé en ce que** R' représente un radical méthyle, éthyle, n-propyle, isopropyle, n-butyle, 2-butyle, isobutyle, pentyle, hexyle, en particulier un radical éthyle, propyle et/ou butyle.

4. Procédé selon au moins l'une des revendications précédentes, **caractérisé en ce que** la réaction est effectuée en présence d'une base de type amine de formule (III)
NR²₃ (III)
dans laquelle R² représente H, un groupe allyle, aryle ou un radical alkyle en C₁-C₁₂ linéaire, cyclique ou ramifié, aryloxy, allyloxy ou alcoxy à fragments alkyle en C₁-C₁₂ linéaires, cycliques ou ramifiés, ou une association des substituants cités.

5. Procédé selon au moins l'une des revendications précédentes, **caractérisé en ce qu'**on ajoute à la solution réactionnelle l'anhydride d'acide alkylphosphonique cyclique soit sous forme de masse fondue, soit dissous dans un solvant.

6. Procédé selon la revendication 5, **caractérisé en ce qu'**on ajoute l'anhydride d'acide alkylphosphonique cyclique dissous dans un solvant aprotique.

7. Procédé selon au moins l'une des revendications précédentes, **caractérisé en ce qu'**avant l'addition de l'anhydride d'acide alkylphosphonique la solution réactionnelle est portée à la température ambiante.

8. Procédé selon au moins l'une des revendications précédentes, **caractérisé en ce que** l'anhydride d'acide alkylphosphonique est utilisé en une quantité allant d'un tiers de la quantité stoechiométrique à au-delà de la quantité stoechiométrique, par rapport au composé de départ.
